# EUROPÄISCHE PATENTSCHRIFT

(11) **EP 4 027 861 B1**
(45) Veröffentlichungstag und Bekanntmachung des Hinweises auf die Patenterteilung: **12.03.2025**
(21) Anmeldenummer: 20764651.4
(22) Anmeldetag: 31.08.2020
(51) Int. Cl.: A61B 3/10

(54) **VORRICHTUNG ZUR MESSUNG VON BIOMETRISCHEN GRÖSSEN DES AUGES**
DEVICE FOR MEASURING BIOMETRIC VARIABLES OF THE EYE
DISPOSITIF POUR MESURER DES GRANDEURS BIOMÉTRIQUES DE L'OEIL

(30) Priorität: 13.09.2019 DE 102019214002
(43) Veröffentlichungstag der Anmeldung: 20.07.2022
(73) Patentinhaber: Carl Zeiss Meditec AG, 07745 Jena (DE)
(72) Erfinder: BÖHME, Beate, 07751 Großpürschütz (DE); BERGNER, Roland, 07745 Jena (DE); EBERSBACH, Ralf, 04626 Schmölln (DE); MÜLLER, Lothar, 07646 Ottendorf (DE)
(74) Vertreter: Kintzel, Klaus-Peter
(86) Internationale Anmeldenummer: PCT/EP2020/074211
(87) Internationale Veröffentlichungsnummer: WO 2021/047933

(56) Entgegenhaltungen:
- EP-A1- 3 692 892
- EP-A2- 3 384 826
- WO-A1-2019/076335
- DE-A1- 102012 019 474
- JP-A- 2016 028 682
- JP-A- 2019 080 867
- US-A1- 2013 141 695
- US-A1- 2017 314 908
- US-B2- 10 085 638

## Beschreibung

Die vorliegende Erfindung betrifft eine Vorrichtung zur Messung von biometrischen Größen des Auges, die insbesondere Eingang in die Berechnung von Intraokular-Linsen finden.

Solche Größen sind die Radien von Vorder- und Rückseite der Kornea mit deren Asphärizität und Dicke, die Radien von Vorder- und Rückseite der Linse und deren Dicke, sowie Vorderkammertiefe und Achslänge des Auges. Relevant sind weiterhin die Lage und Form der optisch wirksamen Grenzflächen bzw. der optisch für das Sehvermögen des Auges relevanten Flächen wie Kornea-Vorder-/Rückseite, Linsenvorder- /und Rückseite und Retina. Diese Grö-ßen oder einige dieser Größen werden für die Berechnung von intraokular Linsen - im Folgenden als IOL-Berechnung bezeichnet - gemäß bekannter IOL-Formeln oder mittels Ray-Tracing-Methoden benötigt.

Im Stand der Technik sind für die Messung der biometrischen Größen des gesamten Auges nur OCT (optical coherence tomography) -Systeme und Topographie/OCT-Kombinationssysteme bekannt. Zwar messen Scheimpflug-, PCI (partial coherence interference) -, Topographie-Systeme oder Kombinationsgeräte derselben einige der oben erwähnten Größen, jedoch können diese Kombinationen nicht alle Parameter des Auges vermessen.

Insbesondere ist eine Vermessung der Linsen-Rückseite und der Retina bzw. des jeweiligen Profils dieser Flächen nicht möglich, da diese Systeme selbst in einem Kombinationssystem auf die Messung der Vorderkammer und der axialen eindimensionalen Länge des Auges beschränkt sind.

WO 2019/076335 offenbart eine OCT-Anordnung mit einem Messmodus für den vorderen Augenabschnitt und einem Messmodus für den hinteren Augenabschnitt (Absätze 64-65). Ein optisches Element (14-16, Figur 9) wird vor dem xy-Scanner angeordnet, um zwischen den Modi umzuschalten (Absatz 107). Beim Umschalten zwischen den Messmodi wird der xy-Scanner konvergent oder mit kollimiertem Licht bestrahlt (Figuren 9-10).

Lediglich die OCT-Systeme mit Messung von Vorder- und Hinterkammer des Auges und Topographie/OCT-Systeme ebenfalls mit Hinter- und Vorderkammermessung sind in der Lage, das gesamte Auge zu vermessen.

Eine weitere mögliche Kombination ist eine Kombination aus Topographie/ Scheimpflug für die Messung der Vorderkammer und OCT für die Hinterkammer. Da das OCT jedoch auch die Vorkammer erfassen kann, ist der Zugewinn durch die Scheimpflug-Vorderkammermessung gegenüber den zusätzlichen Kosten gering.

Gegenüber den Topographie/OCT-Kombinationssystemen haben reine OCT-Systeme den Nachteil, dass die Vermessung der Topographie der Cornea mittels klassischen Topometern (insbesondere Placido-Systemen) wesentlich genauer ist als die durch Bewegungsartefakte beeinflussten Messungen der OCT-Systeme. Zwar können diese Bewegungsartefakte durch schnellere Messungen oder durch genaue Registrierung der Lage des Auges reduziert werden. Jedoch ist dies nur unter erheblichem Aufwand und nicht ohne weiteres zuverlässig genug möglich.

Als ein Topographie/OCT-Kombinationssystem wird z.B. in der US 2004/ 066489 A1 eine Kombination aus Placido-Topographen und einem Time Domain-B-Scan-OCT beschrieben. Damit lässt sich prinzipiell das gesamte Auge biometrisch vermessen. Jedoch zeigt die beschriebene Vorrichtung einige erhebliche Nachteile, die die Zuverlässigkeit der Messwerte herabsetzen. Eine Placido-Topographie ist zwar sehr hochauflösend, aber in der Rekonstruktion der Oberfläche weniger reproduzierbar im Vergleich zu Keratometermessungen. Dies liegt zum einen an den Annahmen bei der Rekonstruktion der Topographie, um die hohe Auflösung zu erreichen, und/oder in der mangelnden Telezentrie/unzureichender Fokussierbarkeit vieler Topographiesysteme im Vergleich zu Keratometern, so dass Positionierungsfehler des Messgerätes gegenüber dem Patienten bei der Topographiemessung relevant werden und die Genauigkeit der Messung reduzieren.

Da bei dieser Lösung Scanner und Auge in beiden Messmodi gleich beleuchtet wird, und keine Mittel zur Veränderung der Divergenz des auf das Auge treffenden Lichtes je nach untersuchter Region am Auge vorhanden sind, entsteht weder im Retina- noch im Cornea-Messmodus ein scharf begrenzter Fokus, und die Ortsauflösung ist nur sehr gering.

Außerdem erlauben Placido-Topographen nicht die Berücksichtigung von sogenannten Skrew-Rays, welche immer dann bei der Beleuchtung von Placido-Ringen entstehen, wenn die Cornea nicht nur in einer medianen Ebene durch den Kornea-Vertex sondern auch in einer Ebene senkrecht dazu, d. h. azimutal gekrümmt ist. Durch die Nichtberücksichtigung wird die Oberfläche der Kornea nicht richtig wiedergegeben. Insgesamt gibt daher ein Placido-Topograph nicht zuverlässig genug den Radius oder allgemein die Vorderseite der Cornea wieder, wie sie für die IOL-Berechnung benötigt wird.

Ferner sind Time-Domain-OCT-Systeme zu langsam und preisakzeptable Spektrometer-basierte Systeme haben entweder nicht die axiale Auflösung und/oder eine zu geringe axiale Scan- oder Erfassungstiefe, so dass die Augenlängenmessung nicht mit der für die IOL-Berechnung notwendigen Auflösung erfolgt oder so erfolgt, dass nur partielle Tiefenmessungen durchgeführt werden. Ganzaugen-Biometrie, d.h. die Ermittlung der optisch für das Sehvermögen des Auges relevanten Flächen des gesamten Auges in ihrer Lage und ihrem Profil im Auge ist jedoch in beiden Fällen durch separate Vermessung der Vorder- und Hinterkammer und anschließende Synthese der Daten prinzipiell möglich, doch unzuverlässig bzw. ungenau.

Daher lässt das Placido-Time-Domain-OCT-System keine für die IOL-Berechnung ausreichend zuverlässige und einfache Gewinnung aller Biometrischen Daten zu.

Als ein Topographie/OCT-Kombinationssystem wird in der US 2005/0203422 A1 eine Kombination aus einfachem Keratometer und einem B-Scan-OCT beschrieben. Auch in diesem System lassen sich wichtige biometrische Größen der Biometrie des Auges bestimmen. Jedoch zeigt auch die beschriebene Vorrichtung einige erhebliche Nachteile, die die Zuverlässigkeit der Messwerte herabsetzen oder wichtige Punkte offenlassen, die für eine Gesamtaugen-Biometrie relevant sind.

Das beschriebene Keratometer lässt lediglich die robuste Messung der Radien der Vorderseite der Cornea zu. Eine Beschreibung der Oberfläche der Kornea höherer Ordnung oder mit einer höheren Auflösung als die des beschriebenen Keratometers ist nicht möglich. Diese wird aber für die Berechnung intraokularer Linsen (kurz IOLs), insbesondere torischer IOLs zunehmend benötigt.

Ferner wird weder das Problem der Zuordnung der mittels Keratometer gemessenen Topographie zu den Orts-Daten der OCT-Daten gelöst, noch wird sichergestellt, dass die OCT-Messungen besonders schnell erfolgen, um die Augenbewegung während der Messung zu kompensieren.

Eine Vorrichtung zur verlässlichen Bestimmung biometrischer Messgrößen des gesamten Auges wird in der DE 10 2012 019474 A1 beschrieben. Die Vorrichtung basiert hierbei auf einem Multipunkt-Keratometer und einer OCT-Anordnung, wobei das Multipunkt-Keratometer so ausgebildet ist, dass die Keratometermesspunkte kollimiert beleuchtet und telezentrisch detektiert werden. Die OCT-Anordnung ist als lateral scannendes Sweptsource-System mit einem das Auge in seiner gesamten axialen Länge umfassenden Erfassungsbereich ausgelegt.

Nachteilig wirkt sich bei dieser Lösung aus, dass die Auslenkung der Scanner im Vorderkammermodus, bedingt durch den Abstand der Scanner zum Auge und durch fehlende Linsen zwischen Scanner und Auge kleiner ist, als deren Auslenkung im Retinamode. Um hohe Winkelauflösung bei gleichzeitig großen Drehwinkelbereichen in beiden Modi sicherstellen zu können, sind eine aufwendige Steuerung, sowie eine äußerst präzise Konstruktion der Scanner erforderlich. Muss der Abstand des/der Scanner zum Auge, auf Grund anderer Beleuchtungseinheiten oder Messfunktionen im Gerät, wie z.B. einer Beleuchtungseinheit für ein Keratometer mit hunderten von Punkten weiter vergrößert werden, verschärft sich die Anforderung an die Winkelauflösung weiter, bis hin zu dem Zustand, dass kommerziell erhältliche Scanner diese Anforderungen nicht mehr erfüllen können.

Weiterhin ist von Nachteil, dass im Retinamodus nur einer der Scanner im richtigen Abstand zur augenseitigen Linse ist, um den Drehpunkt des Strahls in die Augenpupille zu bringen. Somit ist nur ein 1-dimensionaler Retinascan möglich. Im Vorderkammermodus lassen sich zwar 2-dimensionale Scans realisieren, allerdings kann die Strahlauslenkung hierbei nur divergent erfolgen. Das heißt, der Winkel zwischen optischer Achse und Strahl steigt mit der Auslenkung des Scanners, weil die Optik zwischen Scanner und Auge ausgeschwenkt ist.

Die Kombination des OCT -Systems mit den anderen Messfunktionen des Gerätes bedingt einen Mindestabstand der Scanner und Linsen zum Auge, wodurch der Strahldurchmesser auf den Spiegeln der Scanner relativ groß ist. Größere Spiegel sind prinzipiell immer langsamer zu bewegen als kleine Spiegel und damit nachteilig für kurze Messzeiten, die wegen möglicher Augenbewegungen immer wünschenswert sind.

Die Umschaltung zwischen Retina- und Vorderkammermodus wird durch seitliches Schwenken von Optik vor und nach den Scannern realisiert. Bei einer Erhöhung der Scanwinkel erfordert jedoch insbesondere das Ausschwenken der Optik nach dem Scanner erheblichen Bauraum. Weiterhin erfordern die Toleranzen hochgenaue mechanische Anschläge, um die notwendige Abbildungsqualität und eine feststehende Bildmitte während des Schaltvorganges zu erreichen.

Ein weiteres, aus einem Mikroskop und einem OCT-System bestehendes System wird in der DE 10 2015 012 387 A1 beschrieben. Auch bei diesem System sind Messmodi für die Retina und die Vorderkammer vorgesehen, wobei die Umschaltung durch Einschwenken von Linsensystemen erfolgt.

Die Vielzahl der für den Schaltmechanismus erforderlichen Komponenten wirkt sich nachteilig aus. Zudem unterscheidet sich der Abstand zwischen Optik-System und Auge in den beiden Messmodi stark, was in der Anwendung das Handling stark verkompliziert.

Der vorliegenden Erfindung liegt die Aufgabe zugrunde, eine Vorrichtung zu offenbaren, die schnell, zuverlässig, reproduzierbar und mit der nötigen Genauigkeit und Auflösung Messwerte der biometrischen Größen am Auge liefert, die für eine Berechnung von Intraokularlinsen relevant sind. Dabei soll die Messung vorzugsweise auf Ganzaugen-Scans basieren, die die optisch für das Sehvermögen des Auges relevanten Flächen des gesamten Auges in ihrer Lage und ihrem Profil im Auge bestimmen können. Die Vorrichtung soll dabei die Möglichkeit zweier verschiedener Messmodi für die vorderen (Vorderkammer) und hinteren (Retina) Augenabschnitte bieten, wobei für die Umschaltung möglichst wenige Komponenten und ein möglichst geringer Bauraum erforderlich sind, zusätzlich die Lage des Auges gleichbleiben kann und der Scanner in beiden Modi mit ähnlichen Auslenkungen benutzt werden kann.

Diese Aufgabe wird mit der Vorrichtung nach Anspruch 1 zur Messung von biometrischen Größen des Auges zur Berechnung von Intraokularlinsen, bestehend aus einem Multipunkt-Keratometer und einer OCT-Anordnung, die als scannendes Sweptsource-System mit einem das gesamte Auge erfassenden Messbereich ausgelegt ist und über zwei Messmodi verfügt, dadurch gelöst, dass der Scanner des OCT-Sweptsource-Systems ein 2D-Scanner ist, dass zur Variation der Messmodi im Strahlengang zwischen 2D-Scanner und zu vermessendem Auge ein axial verschiebbares optisches Element angeordnet ist und im Strahlengang zwischen 2D-Scanner und dem Interferometer des OCT-Sweptsource-Systems optische Elemente vorhanden sind, die sich in Abhängigkeit vom einzustellenden Messmodus wahlweise im Strahlengang befinden, oder axial verschiebbar sind. Insbesondere können zwei optische Elemente vorhanden sein, die sich wahlweise im Strahlengang befinden.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Durch den Multipunkt-Keratometer wird sichergestellt, dass zum einen genügend viele Keratometerpunkte für eine Vermessung der Kornea-Oberfläche mit hoher Auflösung zur Verfügung stehen. Hingegen wird durch die Telezentrie sichergestellt, dass Positionierunzulänglichkeiten des Messgerätes in Bezug auf das zu vermessenden Auge nicht zu einer örtlichen Fehlzuordnung der Reflexpunkte führen.

Durch den das gesamte Auge in seiner Länge erfassenden Sweptsource-OCT-Scan wird erreicht, dass sowohl Vorderkammer- als auch Retina-Strukturen im A- bzw. B-Scan detektierbar sind und somit eine Orientierung an der Retina und der Kornea (Vorderkammer) möglich wird.

Obwohl die vorgeschlagene Vorrichtung vorzugsweise auf einem Sweptsource-OCT-System basiert, sind auch OCT-Systeme vom Typ Time-Domain, Frequenz-Domain oder Spektral-Domain anwendbar. Allerdings sichert das Sweptsource-OCT besser als andere OCT-Varianten eine schnelle Messung der A-Scans über die gesamte Augenlänge, die zudem frei von Bewegungsartefakten ist.

Die Erfindung wird nachfolgend anhand von Ausführungsbeispielen näher beschrieben. Dazu zeigen:
- Figur 1:: die Prinzipdarstellung der erfindungsgemäßen Vorrichtung,
- Figur 2:: eine bevorzugte Ausgestaltung der erfindungsgemäßen Vorrichtung im Retina-Modus und
- Figur 3:: eine bevorzugte Ausgestaltung der erfindungsgemäßen Vorrichtung im Vorderkammer-Modus.

Die vorgeschlagene Vorrichtung zur scannenden Messung des Auges besteht aus einer OCT-Anordnung mit einem Messmodus für den vorderen und einem Messmodus für den hinteren Augenabschnitt.

Erfindungsgemäß ist zwischen x-y-Scanner und Auge ein optisches Element angeordnet ist, das zur Umschaltung zwischen den Modi axial verschiebbar ist, und dass der x-y-Scanner zur Umschaltung zwischen den Modi konvergent oder divergent beleuchtet wird.

Dabei wird der x-y-Scanner im Retina-Messmodus fokussiert beleuchtet und es erfolgt durch das optische Element eine Abbildung des Scanner-Drehpunktes in die Augenpupille, wobei das optische Element im Vorderkammer-Messmodus zum x-y-Scanner hin verschoben und der Scanner divergent beleuchtet wird.

Einer ersten Ausgestaltung entsprechend besteht die vorgeschlagene Vorrichtung zur Messung von biometrischen Größen des Auges zur Berechnung von Intraokularlinsen aus einem Multipunkt-Keratometer und einer OCT-Anordnung, die als scannendes Sweptsource-System mit einem das gesamte Auge erfassenden Messbereich ausgelegt ist und über zwei Messmodi verfügt.

Erfindungsgemäß ist der Scanner des OCT-Sweptsource-Systems zurAblenkung des OCT-Strahles senkrecht zur optischen Achse als ein 2D-Scanner ausgeführt, wobei dieser aus zwei Scannern für x- und y-Ablenkung mit oder ohne Zwischenabbildung bestehen kann. Vorzugsweise handelt es sich um einen zweiachsigen Scanner (x-y-Scanner) oder MEMS.

Im Gegensatz zum Stand der Technik wird der Scanner mit einem fokussierten Bündel beleuchtet, wobei der Fokus des Beleuchtungslichtes vor oder nach dem Scanner oder den Scannern liegt.

Weiterhin ist erfindungsgemäß zur Variation der Messmodi im Strahlengang zwischen 2D-Scanner und zu vermessendem Auge ein axial verschiebbares optisches Element angeordnet, und sind im Strahlengang zwischen 2D-Scanner und dem Interferometer des OCT-Sweptsource-Systems eine Delay-Line und mindestens ein variables optisches Element vorhanden, das den Fokus des auf den Scanner fallenden Lichtes zur Variation der Messmodi vor oder hinter den Scanner verschiebt. Dieses variable optische Element kann ebenfalls eine axial verschiebbare Linse oder Linsenkombination sein, oder aus zwei Elementen bestehen, die sich in Abhängigkeit vom einzustellenden Messmodus wahlweise im Strahlengang befinden.

Sowohl das verschiebbare als auch die wahlweise einbringbaren optischen Elemente sind hierbei als Linsen oder Linsensysteme ausgeführt. Diese Linsen oder Linsensysteme sind vorzugsweise so dimensioniert, dass deren Abstände und Brennweiten in einem festen Verhältnis zueinanderstehen stehen.

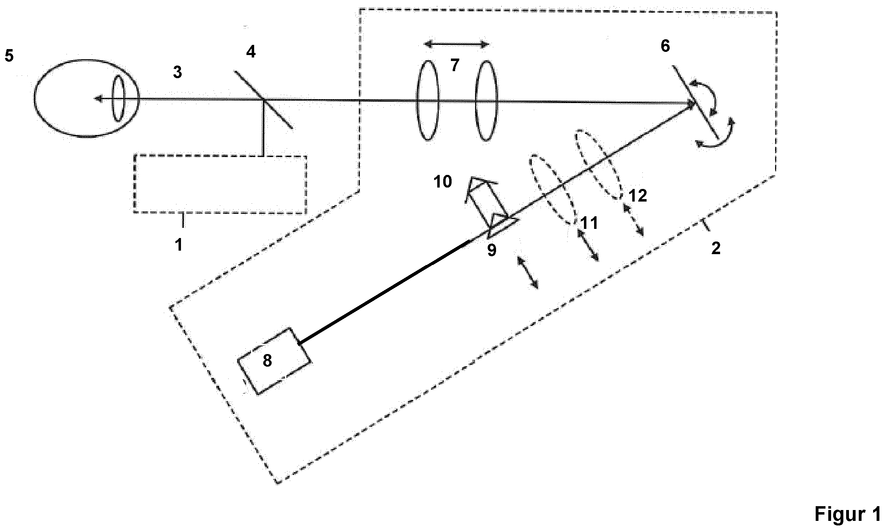

Hierzu zeigt die **Figur 1** eine Prinzipdarstellung der erfindungsgemäßen Vorrichtung basierend auf einem Multipunkt-Keratometer **1,** OCT-Anordnung **2** und einem (nicht dargestellten) Sweptsource-System. Das Multipunkt-Keratometer **1** wird über den, auf der Geräteachse **3** angeordneten Strahlteiler **4** mit dem Strahlengang der OCT-Anordnung **2** vereint. Zur Variation der Messmodi verfügt die OCT-Anordnung **2** im Strahlengang zwischen dem Scanner **6** und dem zu vermessenden Auge **5** eine verschiebbare Linse **7** und im Strahlengang zwischen dem Scanner **6** und dem Interferometer **8** als Delay-Line ein Prisma **9** und einem Umlenk-Winkelspiegel **10,** sowie als variables optisches Element zwei Linsen **11** und **12,** die sich in Abhängigkeit vom einzustellenden Messmodus wahlweise im Strahlengang befinden.

Erfindungsgemäß wird für den Retina-Messmodus die Linse **7** im Strahlengang in Richtung Auge **5,** in eine Scanner-ferne Position verschoben. Zusätzlich befindet sich die Linse **12** im Strahlengang.

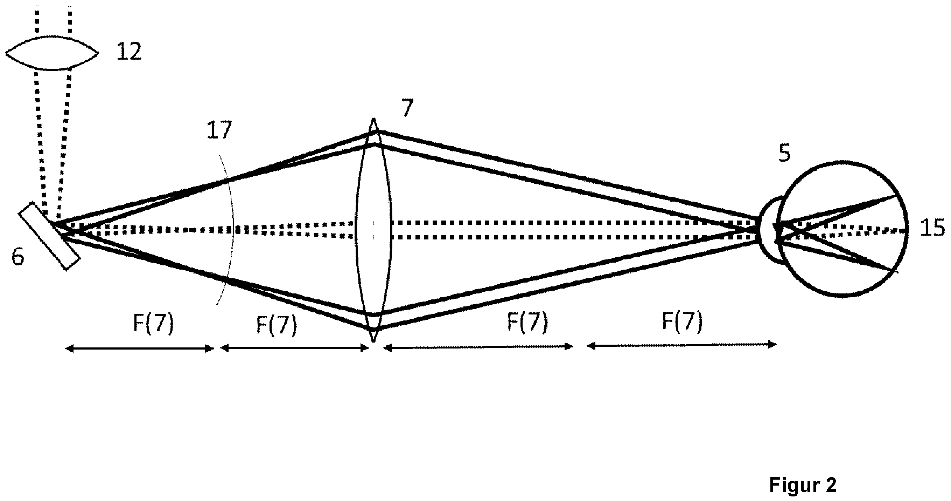

Hierzu zeigt die **Figur 2** eine bevorzugte Ausgestaltung der erfindungsgemäßen Vorrichtung im Retina-Modus. Hierbei entspricht der Abstand der Linse **7** zum Scanner **6** und zum Auge **5** jeweils deren doppelter Brennweite. Es handelt sich in dieser besonders einfachen Ausgestaltung damit um eine Abbildung des Scanners **6** in die Augenpupille mit dem Maßstab M=1 erfolgt. Die Strahlauslenkung am Scanner **6** wird 1:1 in die Auslenkung am Auge **5** übersetzt. Hiervon abweichende Übersetzungen sind durch abweichende Abstände zwischen Scanner **6,** Linse **7** und Auge **5** erreichbar.

Im Retina-Messmodus wird die Linse **7** damit zur Pupillen-Abbildung benutzt, wobei Scanner **6** mit einem Maßstab M1 in die Pupille des Auges **5** abgebildet wird. Gleichzeitig bewirkt die Linse **7,** dass das Licht, das sich mit seinem Fokus auf einer Kreisbahn um den Scanner **6** bewegt, kollimiert auf das Auge **5** trifft, und am Augenhintergrund **15** fokussiert wird. Die Linse **7** bildet damit vom um den Scanner **6** zentrierten Zwischenbild **17** in die Bildebene am Augenhintergrund **15** ab.

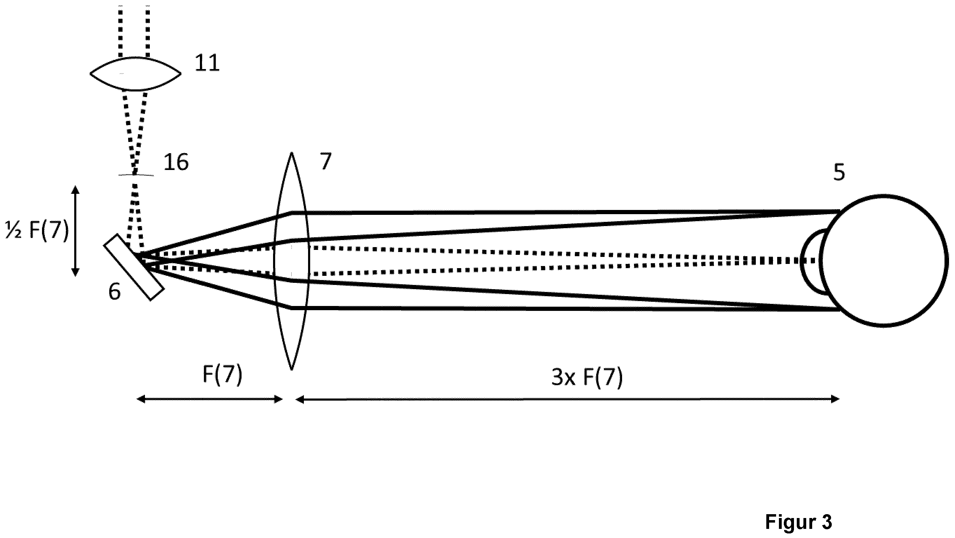

Weiterhin liegt die Kreisbahn, auf der sich der Zwischenfokus (das gewölbte Zwischenbild **17**) während des Scannens bewegt, im Retina-Messmodus zwischen Scanner **6** und Linse **7.** Zwischenbild **17** und Linse 7 sind damit im Vergleich zu **Figur 3** zum Auge **5** hin verschoben. Der Fokus des einfallenden Lichtes, das durch die Linse **12** fokussiert wird, liegt im Abstand der Brennweite F der Linse **7** hinter dem Scanner **6.** Die Bewegung des Scanners **6** beleuchtet damit ein Zwischenbild mit dem Radius r = F und liegt konzentrisch um den Scanner **6.** Der Abbildungsmaßstab der Linse **7** vom Scanner zum Auge **5** beträgt in dieser einfachsten Konfiguration M = 1.

Einer bevorzugten Ausgestaltung entsprechend kann zum Beispiel mit folgenden Parametern im Retina-Messmodus eine Pupillen-Abbildung vom Scanner zum Auge realisiert werden:
- Brennweite der Linse **7** von F=30mm,
- Abstand der Linse **7** vom Auge **5** und vom Scanner **6** von 2F=60mm,
- einem um +/-5 Grad drehbaren Scanner-Spiegel mit einem Durchmesser von ca. 3mm und
- einer Brennweite der Linse **12** von F=120, bei einem Abstand vom Scanner **6** von 90mm.

Mit dieser Ausgestaltung kann ein Strahl am Auge mit dem Durchmesser von 2mm in einem Winkelbereich von +/-10 Grad bewegt werden.

Erfindungsgemäß wird für den Wechsel vom Retina-Messmodus zum Vorderkammer-Messmodus die Linse **7** in Richtung Scanner **6,** in eine Scannernahe Position verschoben. Zusätzlich befindet sich das als Delay-Line dienende Prisma **9** und der Umlenk-Winkelspiegel **10,** sowie die Linse **11** im Strahlengang.

Hierzu zeigt die **Figur 3** eine besonders einfach zu beschreibende und bevorzugte Ausgestaltung der erfindungsgemäßen Vorrichtung im Vorderkammer-Messmodus. Hierbei entspricht der Abstand der Linse **7** zum Scanner **6** deren Brennweite und der Abstand zum Auge **5** deren dreifacher Brennweite.

Der Scanner **6** und die Brennebene der Linse **7** fallen damit zusammen, so dass ein telezentrischer OCT-Strahl am Auge **5** erzeugt wird, der sich während des Scanvorganges parallel zur optischen Achse verschiebt. Weiterhin ist die Linse **11** so angeordnet, dass der Fokus **16** des eingestrahlten Lichtes eine halbe Brennweite vor dem Scanner **6** liegt. Damit erzeugt die Linse **7** nicht nur einen telezentrisch scannenden Strahl, sondern gleichzeitig einen Fokus am Ort des Auges **5,** der im Abstand der 3-fachen Brennweite der Linse **7** liegt.

Dieser Ausgestaltung entsprechend kann zum Beispiel mit folgenden Parametern im Vorderkammer-Messmodus mit gleichem Strahldurchmesser am Scanner, gleicher Auslenkung des Scanners und gleicher Linse **7** am vorderen Augenabschnitt mit einer Apertur von 0,033 in einen Bereich von +/-5,3mm um die optische Achse gescannt werden:
- Brennweite der Linse **7** von F = 30mm,
- Abstand der Linse **7** vom Auge **5** von 3F = 90mm,
- Abstand vom Scanner **6** zur Linse **7** von 1F=30mm,
- einem um +/-5 Grad drehbaren Scanner-Spiegel mit einem Durchmesser von ca. 3mm,
- Prisma **9** und Umlenk-Winkelspiegel **10** im Strahlengang,
- einem Abstand des Fokus **16** vom Scanner **6** von 1/2F = 15mm und
- einer Brennweite der Linse **11** von F=75mm bei einem Abstand vom Scanner **6** von 90mm.

Vorteilhaft kann auch eine geringfügig abweichende Wahl des Abstandes von Linse **7** zum Scanner **6** im Vorderkammermodus sein, so dass die Strahlauslenkung im Vorderkammer-Messmodus divergent, telezentrisch oder konvergent gestaltet werden kann. In **Figure 3** dargestellt sind leicht divergent scannende Strahlen. In Abänderung der oben angegebenen Daten kann dies zum Beispiel durch folgende Abstände realisiert werden:
- Abstand Linse **7** bis Auge **5** von 94,5mm - 3F,
- Abstand Scanner **6** bis Linse **7** von 25,5 - 1F und
- Abstand Fokus **16** bis Scanner **6** von 18,5mm - 1/2F.

Dabei wird der Scanner in Retina- und Vorderkammer-Modus mit gleicher Auslenkung und gleichem Durchmesser benutzt, wobei die Apertur an der Vorderkammer 0.025 und der laterale Scanbereich +-7mm erreicht werden.

In dieser vorteilhaften Ausgestaltung sind die Linsen oder Linsensysteme und Abstände so dimensioniert, dass die Scanwinkel in beiden Messmodi möglichst gleich sind. Hierbei ist zu berücksichtigen, dass die Scanwinkel zum einen so groß sind, dass die gesamte Komea im Vorderkammermodus und zum anderen der volle Sehwinkel im Retinamodus erfasst wird.

In einer weiteren Ausführungsform ist der Scanner kleiner als der Strahldurchmesser am Auge im Retina-Modus. Hierzu wird im Retina-Modus ein Abbildungsmaßstab M > 1 gewählt, z.B. M = 2, so dass der Strahldurchmesser am Scanner 1mm, und am Auge 2mm beträgt. Der Scanwinkel am Auge von +/-5Grad kann dann mit einem Scanner-Drehwinkel von +/-5Grad unter folgenden Bedingungen erreicht werden:
- Abstand Auge **5** bis Linse **7** von 100mm
- Abstand Linse **7** bis Scanner **6** von 50mm
- Brennweite Linse **7** von F = 100/3 = 33,33mm
- Radius des Zwischenbildes **17** von F = 100/6 = 16,66mm.

Der Vorderkammer-Modus mit einem Scanfeld von +/-7mm und einer Apertur von 0,0125 am Auge wird realisiert durch:
- Abstand Auge **5** bis Linse **7** vergrößert auf 119,25mm
- Abstand Linse **7** bis Scanner **6** verkleinert auf 30,75mm und
- Abstand des Fokus **16** zum Scanner **6** von 15,5mm.

Wobei Drehwinkel des Scanners +/-5 Grad und Strahldurchmesser am Scanner 1mm dem Retina-Modus exakt gleich sind. Dies ist erreichbar mit nicht telezentrischem, aber leicht konvergentem Strahlverlauf beim Scannen, wie auch in **Figure 3** gezeichnet. Weiterhin besonders vorteilhaft bei diesem von M=1 abweichenden Maßstab ist der geringere Bauraum bei gleichzeitig vergrößertem Abstand zum Auge.

Die Linse **7** kann erfindungsgemäß auch aus mehreren Teilen bestehen, oder kann sowohl einen festen, als auch einen beweglichen Linsen-Teil enthalten, der zur Umschaltung zwischen den Modi bewegt wird.

Insbesondere sind in den beiden Messmodi folgende Felder zu erreichen:

| | |
|---|---|
| • vorderer Augenabschnitt: | überstreichen der gesamten Kornea, D=10mm bis 20mm |
| • hinterer Augenabschnitt: | 3° bis 10° voller Sehwinkel. |

Weiterhin sind die Brennweiten der Linsen oder Linsensysteme vorzugsweise so zu dimensionieren, dass der Durchmesser des OCT-Beleuchtungsstrahls auf dem Scanner minimiert ist. Dies hat den Vorteil, dass Scanner mit kleinerem Spiegel verwendet werden können, so dass der OCT-Beleuchtungsstrahl schneller bewegt und damit die Dauer der Messung verkürzt werden kann.

Der Erfindung lag die Aufgabe zugrunde, eine Vorrichtung mit zwei verschiedenen Messmodi für die vorderen (Vorderkammer) und hinteren (Retina) Augenabschnitte zu entwickeln, bei der für die Umschaltung möglichst wenige Komponenten und ein möglichst geringer Bauraum erforderlich sind.

Dies wird erfindungsgemäß dadurch erreicht, dass der Fokus des OCT-Strahlenganges im vorderen Augenabschnitt im endlichem Abstand vom System-Austritt bzw. auf den vorderen Augenmedien liegt. Im Gegensatz dazu liegt der Fokus des OCT-Strahlenganges im hinteren Augenabschnitt im Unendlichen, so dass beispielsweise am System-Ausgang annähernd kollimiertes Licht vorliegt, welches dann durch das Auge selbst auf die Netzhaut fokussiert abgebildet wird.

Das Problem des hohen Mechanik-Aufwandes und des Bauraumes wird hier gelöst, indem der Scanner nicht kollimiert, sondern fokussiert oder divergent beleuchtet wird.

Mit der vorgeschlagenen Lösung wird eine Vorrichtung zur Messung von biometrischen Größen des Auges zur Verfügung gestellt, mit der die für eine Berechnung von Intraokularlinsen relevanten biometrischen Größen am Auge mit der nötigen Genauigkeit und Auflösung schnell, zuverlässig und reproduzierbar bestimmt werden können.

Durch Ganzaugen-Scans wird ermöglicht, dass die für das Sehvermögen des Auges relevanten Flächen des gesamten Auges in ihrer Lage und ihrem Profil bestimmbar sind.

Die vorgeschlagene Vorrichtung bietet bei geringem technischen Aufwand und Bauraum zwei verschiedene Messmodi für die vorderen (Vorderkammer) und hinteren (Retina) Augenabschnitte. Dabei bleibt der Abstand des Auges zur Vorrichtung fest, und muss nicht verändert werden.

Bei der vorgeschlagenen Lösung ist es weiterhin von Vorteil, dass der Scanner in den unterschiedlichen Messmodi unterschiedlich beleuchtet wird, so dass im Retina-Messmodus ein Fokus auf der Retina realisierbar ist.

## Patentansprüche

1. Vorrichtung zur scannenden Messung des Auges, bestehend aus einer OCT-Anordnung (2) mit einem Vorderkammer-Messmodus und einem Retina-Messmodus **dadurch gekennzeichnet, dass** zwischen x-y-Scanner (6) und Auge (5) ein optisches Element (7) angeordnet ist, das zur Umschaltung zwischen den Modi axial verschiebbar ist, und dass der x-y-Scanner (6) zur Umschaltung zwischen den Modi konvergent oder divergent beleuchtet wird.

2. Vorrichtung nach Anspruch 1, **dadurch gekennzeichnet, dass** der x-y-Scanner (6) im Retina-Messmodus fokussiert beleuchtet wird, und eine Abbildung des Scanner-Drehpunktes in die Augenpupille durch das optische Element erfolgt, dass das optische Element (7) im Vorderkammer-Messmodus zum x-y-Scanner (6) hin verschoben, und der Scanner divergent beleuchtet wird.

3. Vorrichtung nach den Ansprüchen 1 bis 2, **dadurch gekennzeichnet, dass** das optische Element (7) vor dem x-y-Scanner (6) aus 2 Teilen besteht, von denen mindestens eines axial verschiebbar ausgebildet ist.

4. Vorrichtung nach Anspruch 1, **dadurch gekennzeichnet, dass** sich im Vorderkammer-Messmodus eine erste Linse (7) in einer Scanner-nahen Position und eine Delay-Line (9, 10) und eine zweite Linse (12) im Strahlengang befinden.

5. Vorrichtung nach den Ansprüchen 1 bis 4, **dadurch gekennzeichnet, dass** die erste und zweite Linsen (7, 11 und 12) so dimensioniert sind, dass deren Abstände und Brennweiten in einem festen Verhältnis zueinanderstehen.

6. Vorrichtung nach den Ansprüchen 1 bis 4, **dadurch gekennzeichnet, dass** die erste und zweite Linsen so dimensioniert sind, dass die Scanwinkel in beiden Messmodi möglichst gleich sind.

7. Vorrichtung nach Anspruch 6, **dadurch gekennzeichnet, dass** die Scanwinkel im Vorderkammmer-Messmodus so groß sind, dass die gesamte Komea erfasst wird.

8. Vorrichtung nach Anspruch 6, **dadurch gekennzeichnet, dass** die Scanwinkel im Retina-Messmodus so groß sind, dass der volle Sehwinkel erfasst wird.

9. Vorrichtung nach Anspruch 5, **dadurch gekennzeichnet, dass** die Brennweiten der ersten und zweiten Linsen so dimensioniert sind, dass der Durchmesser des OCT-Beleuchtungsstrahls auf dem Scanner minimiert ist.

## Claims

1. Device for scanning measurement of the eye, consisting of an OCT arrangement (2) with an anterior chamber measurement mode and a retina measurement mode, **characterized**
**in that** an optical element (7) which can be moved axially for switching between the modes is arranged between the x-y scanner (6) and the eye (5), and in that the x-y scanner (6) is illuminated convergently or divergently for switching between the modes.

2. Device according to Claim 1, **characterized in that** the x-y-scanner (6) is illuminated in focused fashion in the retina measurement mode, and the scanner pivot point is imaged into the pupil of the eye through the optical element, **in that** the optical element (7) is displaced towards the x-y-scanner (6) in the anterior chamber measurement mode, and the scanner is illuminated divergently.

3. Device according to Claims 1 to 2, **characterized in that** the optical element (7) in front of the x-y scanner (6) consists of 2 parts, at least one of which is designed to be axially displaceable.

4. Device according to Claim 1, **characterized in that** a first lens (7) in a position in the proximity of the scanner, a delay line (9, 10) and a second lens (12) are situated in the beam path in the anterior chamber measurement mode.

5. Device according to Claims 1 to 4, **characterized in that** the first and second lenses (7, 11 and 12) are dimensioned such that their distances and focal lengths are in a fixed relationship to each other.

6. Device according to Claims 1 to 4, **characterized in that** the first and second lenses are dimensioned such that the scanning angles are as equal as possible in both measurement modes.

7. Device according to Claim 6, **characterized in that** the scanning angles are so large in the anterior chamber measurement mode that the entire cornea is captured.

8. Device according to Claim 6, **characterized in that** the scanning angles are so large in the retina measurement mode that the full viewing angle is captured.

9. Device according to Claim 5, **characterized in that** the focal lengths of the first and second lenses are dimensioned such that the diameter of the OCT illumination beam on the scanner is minimized.

## Revendications

1. Dispositif pour la mesure par balayage de l'œil, composé d'un agencement OCT (2) présentant un mode de mesure de chambre antérieure et un mode de mesure de rétine, **caractérisé**
**en ce qu'**entre le scanner x-y (6) et l'œil (5) est disposé un élément optique (7)qui est axialement mobile pour commuter entre les modes, et **en ce que** le scanner x-y (6) est éclairé de manière convergente ou divergente pour commuter entre les modes.

2. Dispositif selon la revendication 1, **caractérisé en ce que** le scanner x-y (6) est éclairé de manière focalisée dans le mode de mesure de rétine, et une représentation du centre de rotation de scanner dans la pupille de l'œil est effectuée par l'élément optique, **en ce que** l'élément optique (7) est décalé en direction du scanner x-y (6) dans le mode de mesure de chambre antérieure.

3. Dispositif selon les revendications 1 à 2, **caractérisé en ce que** l'élément optique (7) devant le scanner x-y (6) est composé de 2 parties dont au moins l'une est réalisée de manière axialement mobile.

4. Dispositif selon la revendication 1, **caractérisé en ce que** dans le mode de mesure de chambre antérieure, une première lentille (7) se trouve dans une position près du scanner, et une ligne de retard (9, 10) et une deuxième lentille (12) se trouvent dans le chemin optique.

5. Dispositif selon les revendications 1 à 4, **caractérisé en ce que** la première et la deuxième lentille (7, 11 et 12) sont dimensionnées de telle sorte que leurs espacements et focales présentent un rapport fixe les uns par rapport aux autres.

6. Dispositif selon les revendications 1 à 4, **caractérisé en ce que** la première et la deuxième lentille sont dimensionnées de telle sorte que les angles de balayage dans les deux modes de mesure sont identiques dans la mesure du possible.

7. Dispositif selon la revendication 6, **caractérisé en ce que** les angles de balayage dans le mode de mesure de chambre antérieure sont si grands que la cornée entière soit détectée.

8. Dispositif selon la revendication 6, **caractérisé en ce que** les angles de balayage dans le mode de mesure de rétine sont si grands que l'angle de vision entier soit détecté.

9. Dispositif selon la revendication 5, **caractérisé en ce que** les focales de la première et de la deuxième lentille sont dimensionnées de telle sorte que le diamètre du faisceau d'éclairage OCT sur le scanner est minimisé.
